# EUROPÄISCHE PATENTANMELDUNG

(11) **EP 0 610 808 A2**
(43) Veröffentlichungstag der Anmeldung: **17.08.1994**
(21) Anmeldenummer: 94101624.8
(22) Anmeldetag: 03.02.1994
(51) Int. Cl.: C12N 15/57, C12N 9/54, C11D 3/386

(54) **Verbesserte hochalkalische Proteasen**

(30) Priorität: 12.02.1993 DE 4304161
(71) Anmelder: SOLVAY ENZYMES GmbH & Co. KG, D-31582 Nienburg/Weser (DE)
(72) Erfinder: Amory, Antoine, Dr., B-1330 Rixensart (BE); Clippe, André, B-1457 Walaain (BE); Konieczyna-Janda,Gerhard, D-30982 Pattensen (DE); Vetter, Roman, Dr., D-31303 Burgdorf (DE)
(74) Vertreter: Lauer, Dieter, Dr.

(57) **Zusammenfassung**

Beschrieben werden neue, optimierte hochalkalische Proteasen, die sich für die Anwendung in Waschmittelformulierungen eignen. Ebenfalls beschrieben wird ein Verfahren zu ihrer Herstellung, bei dem durch mutierte DNA-Sequenzen transformierte Mikroorganismen eingesetzt werden. Diese DNA-Sequenzen werden gewonnen, indem man DNA-Sequenzen, die für von Bacillus-Spezies produzierte hochalkalische Protease codieren, in bestimmten Positionen durch gerichtete Mutagenese verändert. Es werden hochalkalische Proteasen erhalten, in denen in zwei, drei, vier oder fünf der angegebenen Positionen die ursprünglichen Aminosäuren gegen bestimmte andere Aminosäuren ausgetauscht sind.

## Beschreibung

Die vorliegende Erfindung bezieht sich auf verbesserte hochalkalische Proteasen, bei denen bestimmte Aminosäuren ausgetauscht sind, auf DNA-Sequenzen, die durch gerichtete Mutagenese verändert worden sind und die für diese verbesserten Proteasen codieren, und auf Vektoren, die diese DNA-Sequenzen enthalten. Ebenfalls betrifft die vorliegende Erfindung mit diesen Vektoren transformierte Mikroorganismen, ein Verfahren zur Herstellung von optimierten hochalkalischen Proteasen, sowie Waschmittel, welche diese verbesserten Proteasen enthalten.

Hochalkalische Proteasen sind wertvolle industrielle Produkte mit vorteilhaften Anwendungen, insbesondere in der Waschmittelindustrie, da sie Protein enthaltende Verunreinigungen entfernen. Um wirksam zu sein, müssen diese Proteasen nicht nur proteolytische Aktivität unter Waschbedingungen (pH-Wert, Temperatur) besitzen, sondern müssen darüber hinaus auch mit anderen Waschmittelbestandteilen, d.h. in Kombination mit anderen Enzymen, Tensiden, Gerüststoffen (Builder), Bleichmitteln, Bleichmittelaktivatoren und anderen Zusatz- und Hilfsstoffen verträglich sein, d.h. diesen gegenüber ausreichende Stabilität und in Gegenwart dieser Substanzen ausreichende Wirksamkeit aufweisen.

Hochalkalische Proteasen sind spezielle Enzyme, die gewonnen werden durch Kultivierung von Mikroorganismen, insbesondere durch Kultivierung von Bacillus Spezies, die, wie z. B. Bacillus alcalophilus, die gewünschte hochalkalische Protease produzieren und in das Kulturmedium ausscheiden, aus welchem die Protease isoliert werden kann. Diese hochalkalischen Proteasen unterscheiden sich von gewöhnlichen alkalischen Proteasen, wie sie durch Kultivierung von Bacillus-Spezies, wie insbesondere z. B. B. subtilis, B. amyloliquefaciens und B. licheniformis, gewonnen werden können.

Im Stand der Technik sind eine Reihe von natürlichen und künstlich (gentechnisch) veränderten alkalischen und hochalkalischen Proteasen bekannt. So werden z. B. in den Patentanmeldungen WO 91/00345, EP 328 229 und EP 415 296 Proteasen beschrieben, deren Aminosäuresequenzen durch gezielte Mutagenese (Punktmutationen) an einer Vielzahl von Positionen verändert wurden.

Dennoch besteht weiterhin Bedarf an neuen, weiter optimierten hochalkalischen Proteasen, welche verbesserte Eigenschaften im Hinblick auf ihre Waschleistungen oder Stabilität zeigen.

Es bestand daher die Aufgabe, neue hochalkalische Proteasen mit weiter verbesserten Eigenschaften sowie dafür benötigte DNA-Sequenzen, Vektoren und transformierte Mikroorganismen zur Verfügung zu stellen.

Die Aufgabe wird gelöst durch die erfindungsgemäßen hochalkalischen Proteasen, durch die zugehörigen DNA-Sequenzen, Vektoren und transformierten Mikroorganismen, sowie durch das erfindungsgemäße Verfahren zur Herstellung dieser Proteasen.

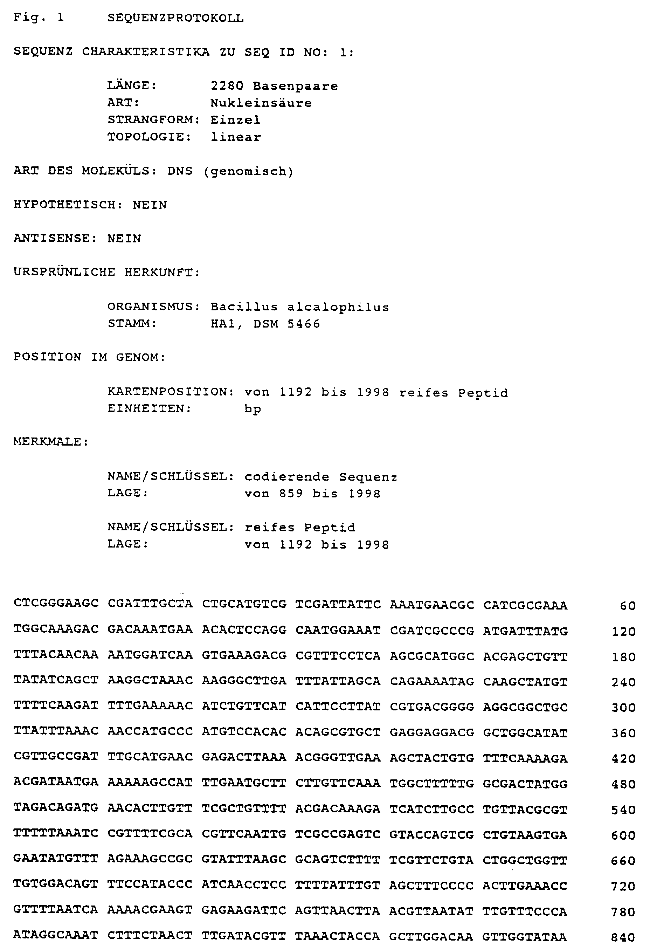

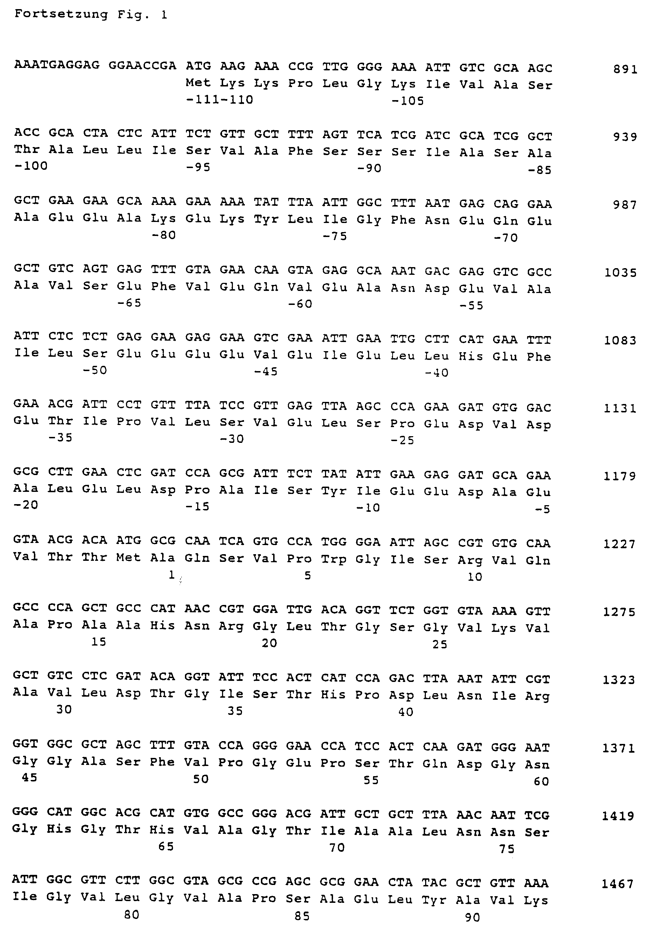

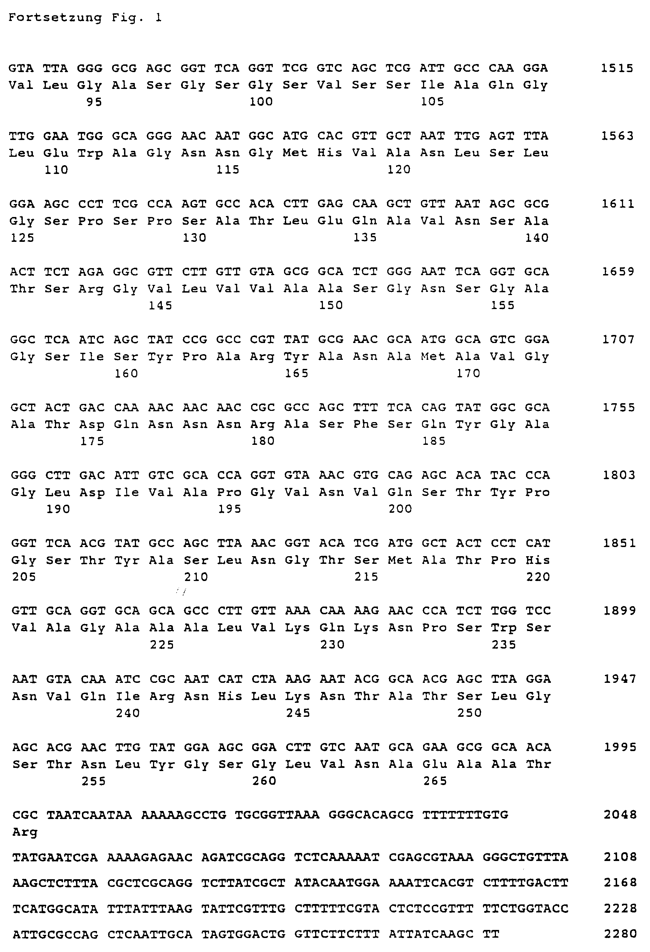

Die Erfindung betrifft hochalkalische Proteasen, welche sich dadurch auszeichnen, daß sie eine Aminosäurensequenz aufweisen, welche wenigstens 80 % Homologie zu der in Fig. 1 angegebenen Aminosäurensequenz besitzt und sich von dieser in zwei, drei, vier oder fünf der Positionen 42, 43, 114, 216, 249 der Fig. 1 oder den dazu homologen Positionen unterscheidet, wobei bei einem Austausch an den betreffenden Positionen
die sich in Position 42 befindliche Aminosäure gegen Arginin,
die sich in Position 43 befindliche Aminosäure gegen Glutamin,
die sich in Position 114 befindliche Aminosäure gegen Arginin,
die sich in Position 216 befindliche Aminosäure gegen Serin, Glutamin oder Alanin,
die sich in Position 249 befindliche Aminosäure gegen Glutaminsäure ausgetauscht ist.

Diese hochalkalischen Proteasen besitzen Molekulargewichte von 26000 bis 28000 g/mol, gemessen durch SDS-Polyacrylamid-Gelelektrophorese gegenüber Referenzproteinen mit bekanntem Molekulargewicht. Ihr pH-Optimum liegt zumeist im Bereich von 8 bis 12,5, wobei unter pH-Optimum derjenige pH-Bereich verstanden wird, in dem die Proteasen maximale proteolytische Aktivität aufweisen. Die erfindungsgemäßen Proteasen weisen eine gute pH-Stabilität auf.

In einer besonderen Ausgestaltung der Erfindung liegen solche Proteasen vor, deren Aminosäurensequenzen eine Homologie von über 90 %, insbesondere aber von über 95 %, zu der Aminosäurensequenz der Fig. 1 besitzen. Unter Homologie zu der in Fig. 1 angegebenen Aminosäurensequenz wird hierbei eine strukturell sehr enge Verwandtschaft der betreffenden Aminosäurensequenzen zu der in Fig. 1 angegebenen Aminosäurensequenz verstanden. Zur Bestimmung der Homologie werden jeweils die einander strukturell entsprechenden Abschnitte der Aminosäurensequenz der Fig. 1 und der damit zu vergleichenden Aminosäurensequenz so miteinander zur Deckung gebracht, daß maximale Strukturübereinstimmung zwischen den Aminosäurensequenzen besteht, wobei durch Deletion oder Insertion einzelner Aminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen") bezogen auf die Gesamtzahl der in der Sequenz der Fig. 1 enthaltenen Aminosäuren gibt dabei die Homologie in % an. Abweichungen in den Sequenzen können sowohl durch Variation, Insertion als auch Deletion von Aminosäuren bedingt sein. Dementsprechend beziehen sich die mit Bezug auf die Fig. 1 bezeichneten Aminosäurepositionen bei den erfindungsgemäßen hochalkalischen Proteasen, die aus zur Fig. 1 wenigstens zu 80 % homologen Proteasen gewonnen wurden, auf die dazu homologen Positionen. Deletionen oder Insertionen in den zu Fig. 1 homologen Proteasen können zu einer relativen Verschiebung der Aminosäurepositionen führen, so daß in homologen Teilstücken von zueinander homologen Aminosäurensequenzen die numerischen Bezeichnungen der einander entsprechenden Aminosäurepositionen nicht identisch zu sein brauchen.

Bei den erfindungsgemäßen hochalkalischen Proteasen müssen die sich in den Austauschpositionen ursprünglich befindlichen Aminosäuren gegen bestimmte andere Aminosäuren ausgetauscht sein. So wird die sich in Position 42 befindliche Aminosäure gegen Arginin, die sich in Position 43 befindliche Aminosäure gegen Glutamin, die sich in Position 114 befindliche Aminosäure gegen Arginin, die sich in Position 216 befindliche Aminosäure entweder gegen Serin, Glutamin oder Alanin und die sich in Position 249 befindliche Aminosäure gegen Glutaminsäure ausgetauscht. Die ursprünglich in den Austauschpositionen vorhandenen Aminosäuren können dabei in zwei, drei, vier oder fünf, vorzugsweise in zwei oder drei der vorgenannten Positionen, ausgetauscht sein. Somit weisen die erfindungsgemäßen Proteasen zwei, drei, vier oder fünf der Aminosäureaustausche N42R, I43Q, N114R, T249E, M216S oder M216Q oder M216A auf, bezogen auf die in Fig. 1 angegebenen Positionen oder einer hierzu homologen Position. Für die Bezeichnung der Mutationen wird hierbei die an sich übliche Nomenklatur verwendet. Die Aminosäuren werden durch den Einbuchstabencode bezeichnet, wobei die urspüngliche Aminosäure der Positionsangabe vorangestellt und die eingeführte Aminosäure der Positionsangabe nachgestellt ist.

Bevorzugt besitzt die erfindungsgemäße hochalkalische Protease eine Aminosäurensequenz, in der die sich in Position 216 befindliche Aminosäure gegen Serin, Glutamin oder Alanin und entweder die sich in Position 43 befindliche Aminosäure gegen Glutamin oder die sich in Position 114 befindliche Aminosäure gegen Arginin oder die sich in Position 249 befindliche Aminosäure gegen Glutaminsäure ausgetauscht ist. Bezogen auf die in Fig. 1 angegebenen oder hierzu homologen Positionen weisen die erfindungsgemäßen Proteasen damit die Aminosäureaustausche M216S oder M216Q oder M216A und entweder I43Q oder N114R oder T249E auf.

Weiter bevorzugt besitzt die erfindungsgemäße hochalkalische Protease eine Aminosäurensequenz in der die sich in Position 216 befindliche Aminosäure gegen Serin, Glutamin oder Alanin und die sich in Position 114 befindliche Aminosäure gegen Arginin und entweder die sich in Position 42 befindliche Aminosäure gegen Arginin oder die sich in Position 43 befindliche Aminosäure gegen Glutamin ausgetauscht ist. Bezogen auf die in Fig. 1 angegebenen oder hierzu homologen Positionen weist die erfindungsgemäße Protease damit die zwei Aminosäureaustausche M216S oder M216Q oder M216A und entweder N42R oder I43Q auf.

Weiter bevorzugt besitzt die erfindungsgemäße hochalkalische Protease eine Aminosäurensequenz, in der die sich in Position 43 befindliche Aminosäure gegen Glutamin und die sich in Position 249 befindliche Aminosäure gegen Glutaminsäure und entweder die sich in Position 114 befindliche Aminosäure gegen Arginin oder die sich in Position 216 befindliche Aminosäure gegen Serin, Glutamin oder Alanin ausgetauscht ist. Bezogen auf die in Fig. 1 angegebenen Positionen oder hierzu homologen Positionen besitzt die erfindungsgemäße Protease damit die drei Aminosäurenaustausche I43Q und T249E und entweder N114R oder M216S oder M216Q oder M216A.

Eine weitere bevorzugte Ausführungsform betrifft eine erfindungsgemäße hochalkalische Protease, die eine Aminosäurensequenz besitzt, in der die sich in Position 43 befindliche Aminosäure gegen Glutamin und die sich in Position 114 befindliche Aminosäure gegen Arginin und die sich in Position 249 befindliche Aminosäure gegen Glutaminsäure und die sich in Position 216 befindliche Aminosäure gegen Serin, Glutamin oder Alanin ausgetauscht ist. Bezogen auf die in Fig. 1 angegebenen Positionen oder hierzu homologen Positionen weist die erfindungsgemäße Protease damit die vier Aminosäureaustausche I43Q, N114R, T249E und M216S oder M216Q oder M216A auf.

Von den vorstehend genannten bevorzugten Austauschkombinationen in der Aminosäurensequenz der erfindungsgemäßen hochalkalischen Proteasen sind ganz besonders die bevorzugt, bei denen die sich in Position 216 befindliche Aminosäure gegen Glutamin ausgetauscht ist. Bezogen auf die in Fig. 1 angegebene Position oder hierzu homologen Position bedeutet dies den einen Aminosäureaustausch M216Q in der Sequenz der erfindungsgemäßen Protease.

In einer weiteren besonders bevorzugten Ausführungsform besitzen die erfindungsgemäßen hochalkalischen Proteasen eine Aminosäurensequenz, in der die sich in Position 114 befindliche Aminosäure gegen Arginin und entweder die sich in Position 43 befindliche Aminosäure gegen Glutamin oder die sich in Position 249 befindliche Aminosäure gegen Glutaminsäure ausgetauscht ist. Bezogen auf die in Fig. 1 angegebenen oder hierzu homologen Positionen weist die erfindungsgemäße Protease damit die zwei Aminosäureaustausche N114R und entweder I43Q oder T249E auf.

Die erfindungsgemäßen hochalkalischen Proteasen sind in ihrer Waschleistung und ihrer Lagerstabilität den Proteasen, in denen an den vorstehend benannten Positionen die Aminosäuren nicht gegen die für jede Position genannten Aminosäuren ausgetauscht wurden, eindeutig überlegen. Dabei ist es überraschend, daß sich gerade mit der Kombination der erwähnten bestimmten Aminosäuren in die vorstehend genannten Positionen der Aminosäurensequenz eine starke Verbesserung in den Eigenschaften im Vergleich mit der Ausgangsprotease erreichen läßt, wobei erstaunlicherweise positive Effekte in der Kombination der genannten Aminosäureaustausche erzielt werden.

Zur Gewinnung der erfindungsgemäßen Proteasen werden Mikroorganismen kultiviert, die mit einem Expressionsvektor transformiert wurden, der das für die betreffende Protease codierende Strukturgen enthält.

Die Erfindung umfaßt daher auch DNA-Sequenzen, die für hochalkalische Proteasen mit den oben beschriebenen Aminosäuresequenzen codieren.

Die erfindungsgemäßen DNA-Sequenzen zeichnen sich dadurch aus, daß sie für eine hochalkalische Protease codieren, die eine Aminosäurensequenz aufweist, welche wenigstens 80 % Homologie zu der in Fig. 1 angegebenen Aminosäurensequenz besitzt und sich von dieser in zwei, drei, vier oder fünf der Positionen 42, 43, 114, 216, 249 der Fig. 1 oder den dazu homologen Positionen dadurch unterscheidet, daß bei einem Austausch an den betreffenden Positionen
die sich in Position 42 befindliche Aminosäure gegen Arginin,
die sich in Position 43 befindliche Aminosäure gegen Glutamin,
die sich in Position 114 befindliche Aminosäure gegen Arginin,
die sich in Position 216 befindliche Aminosäure gegen Serin, Glutamin oder Alanin,
die sich in Position 249 befindliche Aminosäure gegen Glutaminsäure ausgetauscht ist.

Bevorzugt sind solche erfindungsgemäßen DNA-Sequenzen, die für hochalkalische Proteasen codieren, deren Aminosäurensequenzen eine Homologie von über 90 %, insbesondere aber von über 95 %, zu der Aminosäurensequenz der Fig. 1 aufweist und bei denen zwei, drei, vier oder fünf der angegebenen Positionen ausgetauscht sind. Die erfindungsgemäßen DNA-Sequenzen können insbesondere für Proteasen codieren, die die oben angegebenen Aminosäureaustausche in ihrer Sequenz, bezogen auf die in Fig. 1 oder hierzu homologen Positionen, aufweisen.

Weiterhin umfaßt die Erfindung Wasch- und Reinigungszusammensetzungen, die wenigstens eine der erfindungsgemäßen hochalkalischen Proteasen enthalten. Solche Wasch- und Reinigungsmittel können z. B. zur Reinigung von Oberflächen im Hygiene- oder Lebensmittelbereich eingesetzt werden. Bevorzugt werden die erfindungsgemäßen Proteasen in Waschmittelformulierungen zur Reinigung von Textilien oder Geschirr eingesetzt. Für diesen Anwendungszweck stellt die Erfindung eine Gruppe neuer hochalkalischer Proteasen mit einzelnen gegenüber vorbekannten Proteasen verbesserten Eigenschaften, insbesondere verbesserten Waschleistungen und/oder einer verbesserten Enzymstabilität zur Verfügung, aus welcher je nach den speziell benötigten Eigenschaften (Waschleistung, Temperaturbeständigkeit, Verträglichkeit mit anderen Bestandteilen) der einzusetzenden hochalkalischen Protease, eine für die jeweilige spezielle Waschmittelformulierung besonders geeignete erfindungsgemäße Protease ausgewählt werden kann. Die erfindungsgemäßen Proteasen können in Waschmittelformulierungen z. B. in Pulverwaschmitteln, Kompaktwaschmitteln oder Flüssigwaschmitteln einzeln oder gewünschtenfalls auch in Kombination miteinander, gegebenenfalls auch in Kombination mit Waschmittelproteasen des Standes der Technik oder anderen an sich üblichen Waschmittelenzymen, wie z. B. Amylasen, Lipasen, Pektinasen, Nukleasen, Oxidoreduktasen etc., eingesetzt werden. Die erfindungsgemäßen Proteasen werden in den Waschmittelformulierungen in an sich für Waschmittelenzyme üblichen Mengen, insbesondere in Mengen von bis zu 5 Gew.-% (bezogen auf die Trockensubstanz der Gesamtzusammensetzung), vorzugsweise in einer Menge von 0,2 bis 1,5 Gew.-%, verwendet.

Außer den bereits erwähnten Wachmittelenzymen können die Waschmittel der Erfindung alle an sich im Stand der Technik üblichen Waschmittelinhaltsstoffe wie Tenside, Bleichmittel oder Buildersubstanzen (Gerüststoffe), sowie weitere übliche Hilfsstoffe für die Formulierung von Waschmitteln in an sich üblichen Mengen enthalten. Zu den Hilfsstoffen gehören z. B. Verstärker, Enzymstabilisatoren, Schmutzträger und/oder Kompatibilisierungsmittel, Komplex- und Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen.

So enthalten erfindungsgemäße Waschmittelformulierungen in typischer beispielhafter Zusammensetzung, bezogen auf Trockensubstanz,
a) wenigstens 1 Gew.-% eines Tensids oder Tensidgemisches,
b) bis zu 40 Gew.-% eines Builders oder eines Builder-Gemisches,
c) bis zu 40 Gew.-% eines Bleichmittels oder Bleichmittelgemisches, vorzugsweise ein Perborat wie Natriumperborat-Tetrahydrat, Natriumperborat-Monohydrat oder Natriumpercarbonat,
d) bis zu 3 Gew.-% wenigstens einer erfindungsgemäßen Protease und
e) weitere Bestandteile wie Hilfsstoffe etc. ad 100 Gew.-%.

Besonders gut eignen sich die erfindungsgemäßen Proteasen auch für pulverförmige Geschirr-Reinigungsmittel, wie sie z. B. üblicherweise für die maschinelle Geschirreinigung eingesetzt werden.

Pulverförmige Waschmittelformulierungen können in an sich üblicher Weise formuliert werden. Die erfindungsgemäßen Proteasen können dazu, z. B. in Pulver- oder Kompaktwaschmitteln, in Form von Granulaten, Prills oder Pellets, gegebenenfalls auch mit Oberflächenüberzügen versehen, mit den anderen Komponenten der Waschmittelformulierung in an sich bekannter Weise vermischt werden.

Aufgrund ihrer verbesserten Stabilität lassen die erfindungsgemäßen Proteasen sich sehr gut auch in Flüssigwaschmitteln einsetzen.

Die Erfindung umfaßt ferner ein Verfahren zur Herstellung einer erfindungsgemäß optimierten hochalkalischen Protease mit einem transformierten Mikroorganismus, der einen Vektor mit einer DNA-Sequenz enthält, die für eine Aminosäurensequenz codiert, welche wenigstens 80 % Homologie zu der in Fig. 1 angegebenen Aminosäurensequenz der Ausgangsprotease besitzt und sich von dieser in zwei, drei, vier oder fünf, vorzugsweise zwei oder drei der Positionen 42, 43, 114, 216, 249 der Fig. 1, unterscheidet, wobei die sich in Position 42 befindliche Aminosäure gegen Arginin, die sich in Position 43 befindliche Aminosäure gegen Glutamin, die sich in Position 114 befindliche Aminosäure gegen Arginin, die sich in Position 216 befindliche Aminosäure gegen Serin, Glutamin oder Alanin, die sich in Position 249 befindliche Aminosäure gegen Glutaminsäure ausgetauscht ist. Der transformierte Mikroorganismus wird kultiviert und aus dem Kulturmedium die verbesserte hochalkalische Protease isoliert.

Zur Erzeugung der im Herstellverfahren eingesetzten Mikroorganismen kann so vorgegangen werden, daß man
a) zunächst aus einem geeigneten Bakterium, welches eine hochalkalische Protease mit einer Aminosäurensequenz mit mindestens 80 %, vorzugsweise über 90 %, insbesondere aber über 95 % Homologie zu der Aminosäurensequenz der Fig. 1 produziert, die für die Protease codierende DNA-Sequenz (d. h. das Strukturgen der Protease) isoliert,
b) die Nukleotidabfolge dieser DNA-Sequenz bestimmt,
c) in der nunmehr bekannten DNA-Sequenz solche Mutationen (Punktmutationen) erzeugt, daß die mutierte DNA-Sequenz nun für eine hochalkalische Protease, in der Aminosäuren der Ursprungsprotease in vorstehend gegebenen Positionen gegen eine für die betreffende Position genannte Aminosäure ausgetauscht sind, codiert.
d) nachfolgend mit Hilfe der mutierten DNA-Sequenz einen Expressionsvektor erzeugt und
e) den erhaltenen Expressionsvektor in einen geeigneten Mikroorganismus, welcher schließlich zur Herstellung der mutierten hochalkalischen Protease eingesetzt werden kann, transformiert.

Die Strukturgene, die für Aminosäurensequenzen hochalkalischer Proteasen mit wenigstens 80 % Homologie zu der in Fig. 1 angegebenen Aminosäurensequenz codieren, können nach an sich bekannten Methoden erhalten werden. Hierzu wird z. B. aus einem Bakterium ("Donor-Bakterium"), insbesondere aus einer Bacillus-Spezies, die die hochalkalische Protease produziert, die chromosomale DNA isoliert und mit geeigneten Restriktionsendonukleasen partiell hydrolysiert.

Die erhaltenen Restriktionsfragmente der Donor-DNA können z. B. durch Gelelektrophorese, nach Größe aufgetrennt und die Fragmente gewünschter Größe dann mit einer geeigneten Vektor-DNA rekombiniert werden. Zweckmäßig wird als Vektor ein Plasmid verwendet, mit dem die Expression der in den verwendeten Wirtsorganismus eingebrachten Fremd-DNA möglich ist.

Mit der in vitro rekombinierten DNA (Vektor + Restriktionsfragmente der Donor-DNA) können Bakterien, vorzugsweise eine Bacillus-Spezies, transformiert und die Transformanten nach der bekannten Markereigenschaft der Vektor-DNA (z. B. Neomycin-Resistenz) selektiert werden. Unter den so erhaltenen Transformanten können solche, die vermehrt die hochalkalische Protease ausscheiden, auf proteinhaltigen Platten gesucht und danach isoliert werden. Aus einem derartigen Klon wird schließlich die in dieser Transformante eingeführte Plasmid-DNA isoliert. Anschließend wird das Plasmid, welches das Strukturgen der Ausgangsprotease und zusätzliche DNA-Sequenzen aus der Donor DNA-Sequenz umfaßt, mit einer Anzahl verschiedener Restriktionsendonukleasen geschnitten (restingiert), die erhaltenen DNA-Fragmente durch Gelelektrophorese nach Größe getrennt und anhand des gefundenen Bandenmusters eine Restriktionskarte erstellt. Die Kenntnis der Restriktionskarte des Plasmids ermöglicht es, aus diesem durch Schneiden mit ausgewählten Restriktionsendonukleasen ein DNA-Fragment aus der Donor-DNA-Sequenz herauszuschneiden, welches im wesentlichen nur noch das Strukturgen für die hochalkalische Protease, die zugehörigen Pre- und Pro-Einheiten, sowie die für die Genexpression benötigte Promotor-Einheit umfaßt.

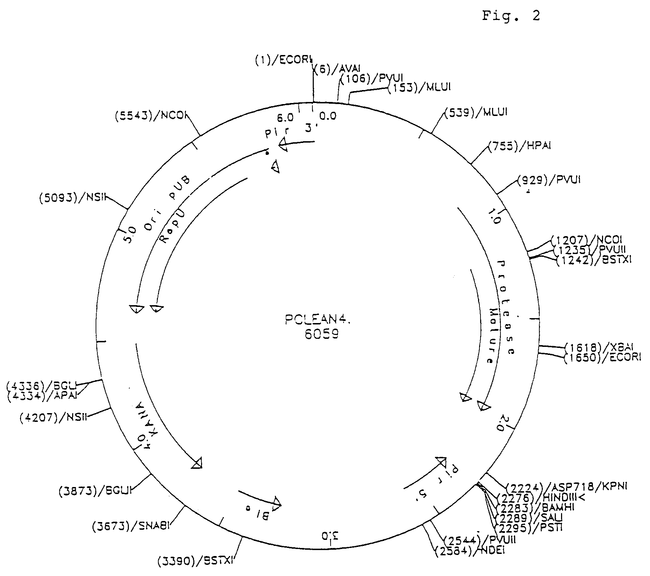

Durch den Wiedereinbau dieser in der Größe reduzierten Donor-DNA-Sequenz in einen geeigneten Vektor kann ein neuer, replizierbarer Vektor erhalten werden, dessen Fähigkeit zur Expression der hochalkalischen Ausgangsprotease überprüft werden kann, indem man ein Bakterium, insbesondere eine Bacillus-Spezies, mit diesem Vektor transformiert, die erhaltene Transformante kultiviert und auf Proteaseaktivität überprüft. Ein Beispiel für einen solchen reduzierten Vektor mit der Bezeichnung pCLEAN4 gibt die Restriktionskarte der Fig. 2 wieder.

Zur Sequenzierung des Proteasestrukturgens wird zunächst der vorstehend beschriebene Vektor in einem geeigneten Mikroorganismus repliziert, und anschließend das Proteasegen isoliert. Dieses wird sodann nach allgemein bekannten Methoden zur DNA-Sequenzierung sequenziert (z. B. nach der Methode von Maxam und Gilbert, 1980, Methods in Enzymology, Grossmann L., Moldave K., eds., Academic Press Inc., New York und London, Vol. 65, 499 oder nach der Dideoxy-Kettenterminator-Methode nach Sanger und Brownlee 1977, Proc. Natl. Acad. Sci. USA 74: 5473).

Die ermittelte Nukleotidsequenz kann nunmehr mit Hilfe des genetischen Codes in die Aminosäurensequenz der Protease übersetzt werden.

Erfindungsgemäß wird die für die Protease codierende DNA-Sequenz durch Austausch der entsprechenden Codons in der Weise mutiert, daß die mutierte DNA-Sequenz für eine optimierte hochalkalische Protease codiert, die eine Aminosäurensequenz besitzt, in welcher in zwei, drei, vier oder fünf, vorzugsweise zwei oder drei der Positionen 42, 43, 114, 216, 249 der Aminosäurensequenz in Fig. 1 oder in einer der dazu homologen Positionen die betreffende Aminosäure gegen eine der vorstehend genannten Aminosäuren ausgetauscht ist.

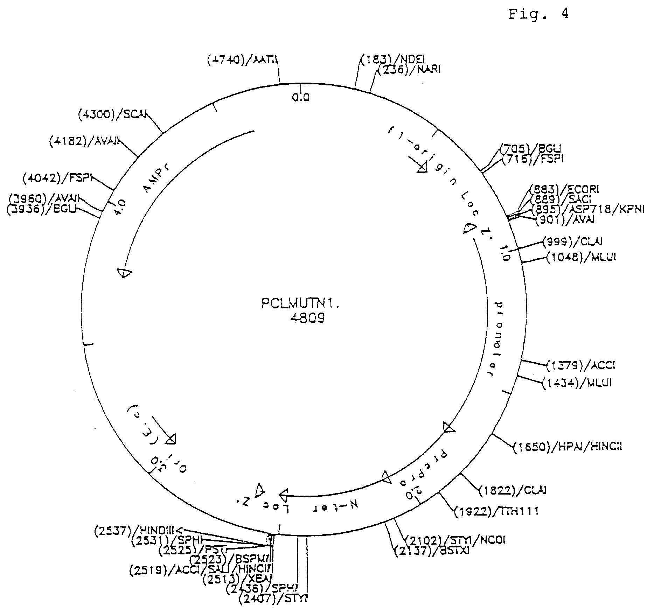

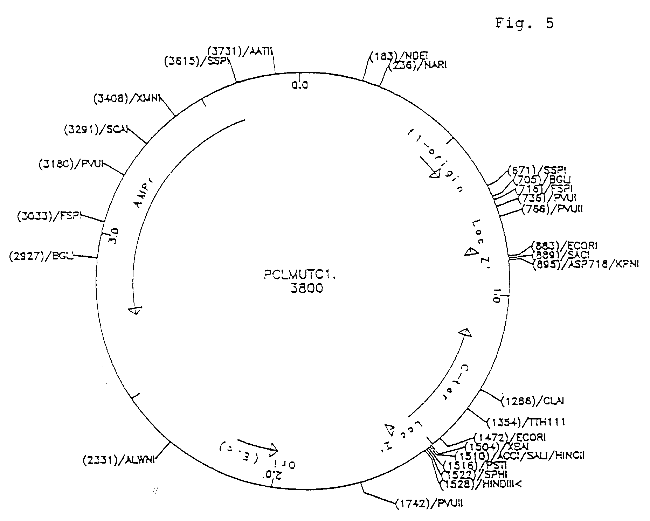

Die Einführung der Punktmutationen in die für die hochalkalischen Protease codierende DNA wird durch bekannte Methoden zur gerichteten Mutagenese durchgeführt. Hierzu wird aus geeigneten Vektoren (Phagemide), z. B. aus pCLMUTN1 der Fig. 4 oder pCLMUTC1 der Fig. 5, gegebenenfalls unter Mithilfe eines Helfer-Phagen, ringförmige Einzelstrang-DNA erzeugt, die die gesamte für die Protease codierende DNA-Sequenz oder vorzugsweise nur die DNA-Sequenz, in welcher die Mutation vorgenommen werden soll, enthält. Mit dieser ringförmigen Einzelstrang-DNA hybridisiert man ein synthetisches, hybridisierfähiges Oligonukleotid, welches in der gewünschten Punktmutationsstelle einen Nukleotidbaustein enthält, der so ausgewählt ist, daß das zugehörige Codon für eine der für die jeweilige Position bereits angegebenen Aminosäuren, z. B. für Arginin in der Position 114, codiert. Zusätzlich ist das Oligonukleotid gegenüber der zu hybridisierenden, originären Nukleotidsequenz noch derart durch einen oder einige wenige weitere Nukleotidbausteine abgewandelt, daß die Codierung der originären Aminosäurensequenz zwar im Rahmen der Degeneration des genetischen Codes erhalten bleibt, jedoch in der originären Nukleotidsequenz eine gegebenenfalls vorhandene Restriktionsstelle im synthetischen Oligonukleotid entfernt bzw. eine weitere Restriktionsstelle in das synthetische Oligonukleotid eingeführt wird. Die entfernte bzw. eingeführte Restriktionsstelle dient später zur Identifizierung der mutierten DNA-Sequenz gegenüber der Ausgangstyp-DNA-Sequenz mit Hilfe geeigneter Restriktionsendonukleasen. In einer Variante wird im Verfahren der gerichteten Mutagenese uracylierte Einzelstrang-DNA als Matrize erzeugt und für die Hybridisierung mit den synthetischen Oligonukleotiden verwendet. Nach Beendigung der Reaktionen des Verfahrens der gerichteten Mutagenese kann der Uracil-haltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann sowohl mit dem isolierten Enzym als auch mit Hilfe eines geeigneten Mikroorganismus mit Uracil-N-Glycosylase-Aktivität durchgeführt werden, der mit mutierter Vektor-DNA transformiert wurde.

Die Ergänzung der durch Hybridisierung erhaltenen partiell doppelsträngigen DNA-Sequenz zum vollständigen Doppelstrang wird dann durch Zugabe der benötigten Nukleotide und unter Einwirkung von DNA-Polymerase und DNA-Ligase durchgeführt. Die erzeugte ringförmige, doppelsträngige DNA-Sequenz wird nachfolgend als Vektor in einen geeigneten Mikroorganismus transformiert. Anschließend werden die mutierten DNA-Sequenzen über die unitären Restriktionsendonukleasen-Erkennungsstellen identifiziert und aus der Transformante isoliert. Wird uracylierte Einzelstrang-DNA eingesetzt, so wird die Replikation z. B. in einem E. coli-Stamm vorgenommen, der vorzugsweise den mutierten, nicht-uracylierten DNA-Strang des im Mutationsverfahren erzeugten Doppelstrang-Vektors vermehrt. Hierdurch wird die Selektion der mutierten DNA-Vektoren zusätzlich erleichtert.

Die für die gerichtete Mutagenese benötigten synthetischen Oligonukleotide werden nach an sich bekannten Methoden hergestellt (z. B. nach Beaucage S.L. und Caruthers M.H., 1981, Tetrahedron Letters 22: 1859-1862).

Die durch die gerichtete Mutagenese erhaltenen ringförmigen, doppelsträngigen DNA-Sequenzen mit den eingeführten Mutationen stellen mutierte Vektoren dar, aus denen durch Behandlung mit geeigneten Restriktionsendonukleasen je nach Fall das gesamte mutierte Protease-Strukturgen oder das mutierte Teilstück des Protease-Strukturgens herausgeschnitten und in einen geeigneten Expressionsvektor eingebracht (subkloniert) werden kann. Mit diesem Expressionsvektor werden dann geeignete Mikroorganismen, z. B. Bacillus-Spezies, transformiert, die nachfolgend zur Expression und Gewinnung der mutierten hochalkalischen Proteasen unter geeigneten Bedingungen kultiviert werden.

In einer bevorzugten Ausgestaltung der Erfindung wird nicht das gesamte Strukturgen für die gerichtete Mutagenese eingesetzt, sondern nur ein Teilstück desselben, in dem die Mutation erzeugt werden soll. Hierzu wird aus dem Vektor der zur Replikation der Strukturgene dient, z. B. die N-terminale oder C-terminale Hälfte des Strukturgens mit geeigneten Restriktionsendonukleasen herausgeschnitten und in einen passenden Phagemiden subkloniert. Man erhält so Vektoren, die entweder die N-terminale oder die C-terminale Hälfte des Strukturgens enthalten und die in einem geeigneten Mikroorgagnismus, z. B. E. coli, zunächst ausreichend repliziert und dann der oben beschriebenen, gerichteten Mutagenese zugeführt werden. Die Mutagenese von DNA-Teilstücken des Strukturgenes hat den Vorteil, daß kürzere Einzelstrang-DNA-Sequenzen verwendet werden können und somit nach dem Hybridisierungsschritt mit synthetischen Oligonukleotiden im partiellen DNA-Doppelstrang wesentlich weniger Nukleotide als bei Verwendung der gesamten DNA-Sequenz zu ergänzen sind. Hierdurch wird der synthetische Aufwand und zusätzlich die Gefahr unerwünschter zufälliger Mutationen reduziert.

Die mutierten DNA-Sequenzen können aus dem zur Erzeugung der Mutationen dienenden Vektoren durch geeignete Restriktionsendonukleasen herausgeschnitten und in entsprechende Restriktionsstellen besitzende Vektoren eingebaut werden, die Vorläufer der eigentlichen, für die Expression der hochalkalischen Protease benötigten Expressionsvektoren darstellen. Diese Vektoren sind so aufgebaut, daß sie außer den geeigneten Restriktionsstellen (z. B. aus einem synthetischen Linker) auch bereits die für die Proteaseexpression in einem Wirtsorganismus benötigten regulatorischen Sequenzen, Signalsequenzen, Promotorsequenzen und die für die Pre- und Proeinheiten der Protease codierenden DNA-Sequenzen enthalten.

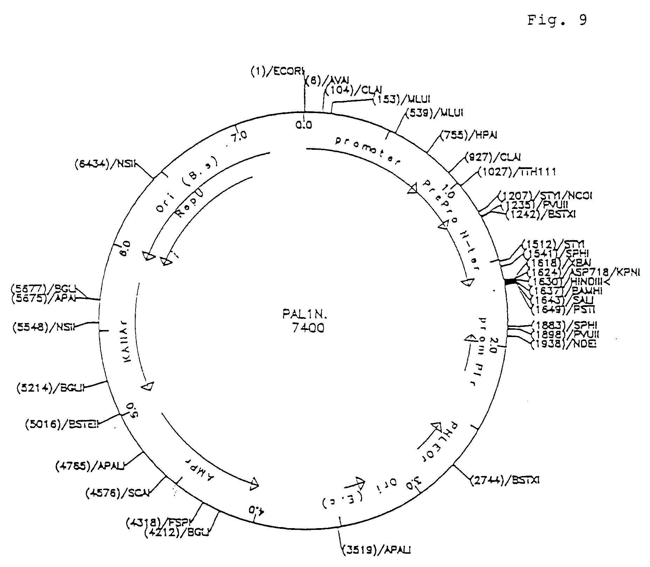

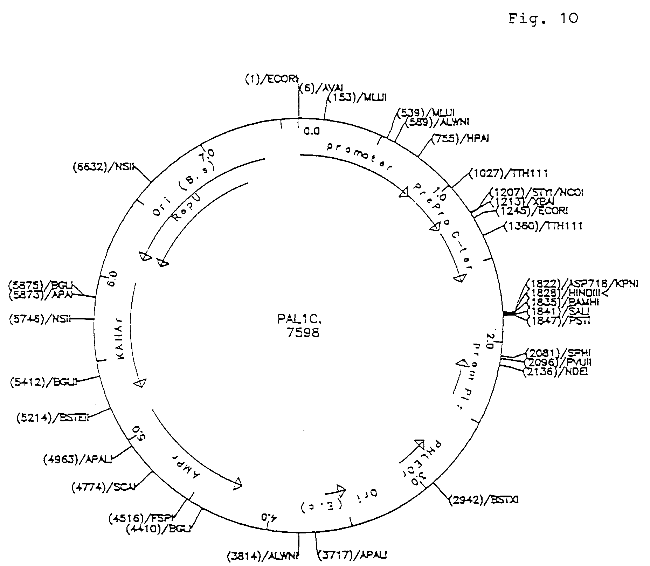

Durch die Subklonierung einer mutierten DNA-Sequenz in einen solchen Vektor wird der eigentliche Expressionsvektor für eine optimierte hochalkalische Protease erhalten. Der Einbau der mutierten DNA-Sequenz in diesen Vorläufer des Expressionsvektors erfolgt so, daß ein Expressionsvektor mit geeignetem Leseraster entsteht. Hierbei können mutierte Teilstücke der für die Protease codierenden DNA-Sequenz, z. B. ein C-terminales oder ein N-terminales Teilstück, in bereits das jeweils restliche nicht mutierte oder ggf. auch mutierte (Erzeugung von Mehrfachmutationen) Teilstück enthaltende Vektoren eingebaut werden; oder es wird die gesamte für die Protease codierende mutierte DNA-Sequenz in Vektoren, welche noch keine Teilstücke dieser Protease-DNA-Sequenz enthalten, eingebaut. Beispiele für solche, bereits Teilstücke des Protease-Gens enthaltende Vorläufervektoren eines Expressionsvektors sind die Vektoren mit der Bezeichnung pAL1N und pAL1C, deren Restriktionskarte in den Fig. 9 bzw. Fig. 10 wiedergegeben sind.

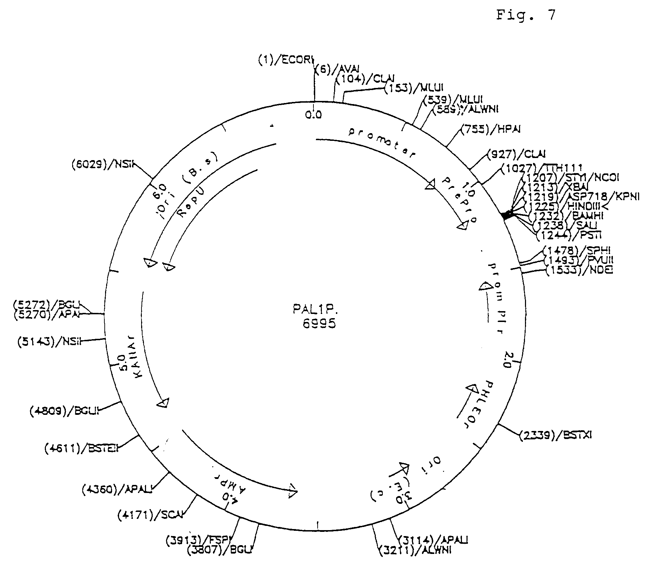

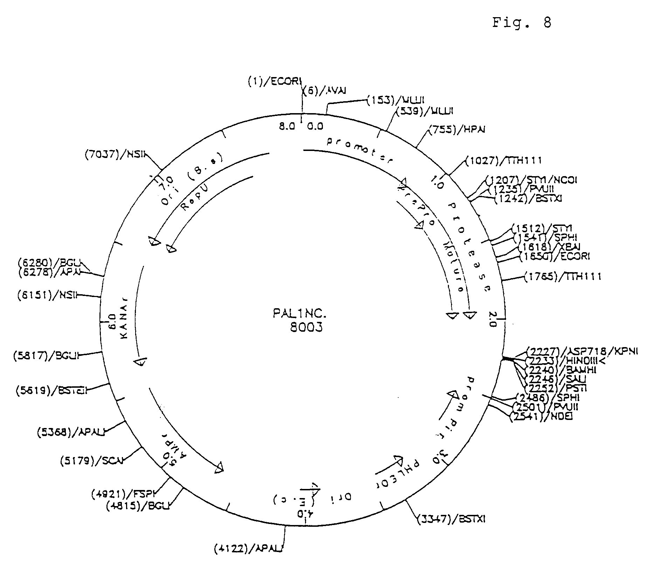

Die Expressionsvektoren-Vorläufer für die bevorzugte Variante der Erfindung (Mutation in der N-terminalen Hälfte oder in der C-terminalen Hälfte) können z. B. erhalten werden, indem man ein Bacillus-Plasmid an einer geeigneten Stelle restringiert und mit einem E. coli-Plasmidfragment, welches Marker und für die Replikation wichtiger Sequenzteile enthält, rekombiniert. Anschließend werden gegebenenfalls solche Restriktionsstellen z. B. durch gerichtete Mutagenese entfernt, die spätere Verfahrensschritte stören würden. Aus dem so erhaltenen Plasmid wird ein neuer Vektor konstruiert, der die aus dem Bacillus-Plasmid und dem E. coli-Plasmid zur Replikation dienenden DNA-Sequenzen, DNA-Sequenzen für den Promotor, DNA-Sequenzen, die für die Pre-Prosequenz der Protease codieren (erhalten aus z. B. dem Plasmid pCLEAN4 der Fig. 2), und einen synthetischen Linker enthält. Ein Beispiel für ein solches Plasmid mit der Bezeichnung pAL1P gibt die Restriktionskarte der Fig. 7 wieder. Der synthetische Linker ist dabei derart ausgewählt, daß nach Schneiden mit geeigneten Restriktionsendonukleasen der Vektor mit der gesamten ursprünglichen oder mutierten DNA-Sequenz, die für die reife Protease codiert, bzw. mit mutierten oder nicht mutierten Teilstücken des Strukturgens kombiniert werden kann. Zur Herstellung eines Expressionsvektor-Vorläufers, der z. B. mit einer mutierten N-terminalen Hälfte des Strukturgens rekombiniert werden soll, wird in den vorstehend konstruierten Vektor, der die genannten Bacillus-, E. coli-, die Promotor- und die Pre- und Prosequenzen der Protease sowie den synthetischen Linker enthält, durch Schneiden des synthetischen Linkers z. B. zunächst die nicht mutierte oder ggf. auch eine bereits mutierte C-terminale Hälfte des Strukturgens eingeführt. Anschließend wird durch nochmaliges Schneiden des synthetischen Linkers die noch fehlende, mutierte N-terminale Hälfte des Protease-Strukturgens eingeführt. Auf diese Weise erhält man einen Vektor vom Typ pAL1NC der Fig 8. Analoges gilt für den umgekehrten Fall. Es wird dann zunächst die nicht mutierte oder ggf. auch eine bereits mutierte N-terminale Hälfte in einen Vektor eingeführt und in den so erhaltenen Vektor später die mutierte C-terminale Hälfte eingebaut, wobei man ebenfalls einen Vektor vom Typ pAL1NC der Fig. 8 erhält.

Mit den oben beschriebenen Expressionsvektoren werden geeignete Bakterien, vorzugsweise Bacillus-Spezies, insbesondere Bacillus subtilis, B. licheniformis oder B. alcalophilus, transformiert. Die Transformanten werden anschließend in an sich bekannter Weise kultiviert und die gebildete hochalkalische Protease aus dem Kulturmedium isoliert. Die Expressionsvektoren können hierzu sowohl in Bakterien, die noch zur Bildung von Eigenprotease befähigt sind, als auch in Protease-defiziente Bakterien (die keine Eigenprotease mehr bilden) transformiert werden. Bei Wirtsorganismen mit Eigenproteasebildung kann die erfindungsgemäße hochalkalische Protease gewünschtenfalls durch anschließende Reinigungsoperationen, z. B. durch Hochauflösende Flüssigchromatographie (HPLC), von den gebildeten Eigenproteasen befreit werden. Ein solcher Reinigungsschritt kann demgegenüber bei Protease-defizienten Wirtsorganismen entfallen, da diese nur (oder im wesentlichen nur) die erfindungsgemäße Protease zu bilden vermögen.

Die folgenden Beispiele sollen die Erfindung erläutern ohne sie jedoch in ihrem Umfang zu beschränken.

Sofern nicht anders angegeben, wurde generell nach Methoden gearbeitet, wie sie in Maniatis et al. (Maniatis et al. = T. Maniatis, E.F. Fritsch, J. Sambrook, Molecular Cloning, A Laboratory Manual, Cold Spring Harbor Laboratory, 1982) beschrieben sind.

Hier verwendete Ausgangsvektoren sind käuflich und auf unbeschränkter Basis verfügbar; oder sie können nach an sich bekannten Methoden aus verfügbaren Vektoren hergestellt werden.

Der im Beispiel 1 eingesetzte und als Bacillus alcalophilus HA1 benannte Bacillus alcalophilus-Stamm ist bei der Deutschen Sammlung von Mikroorganismen (DSM) mit der DSM-Nummer 5466 am 28. Juli 1989 hinterlegt worden.

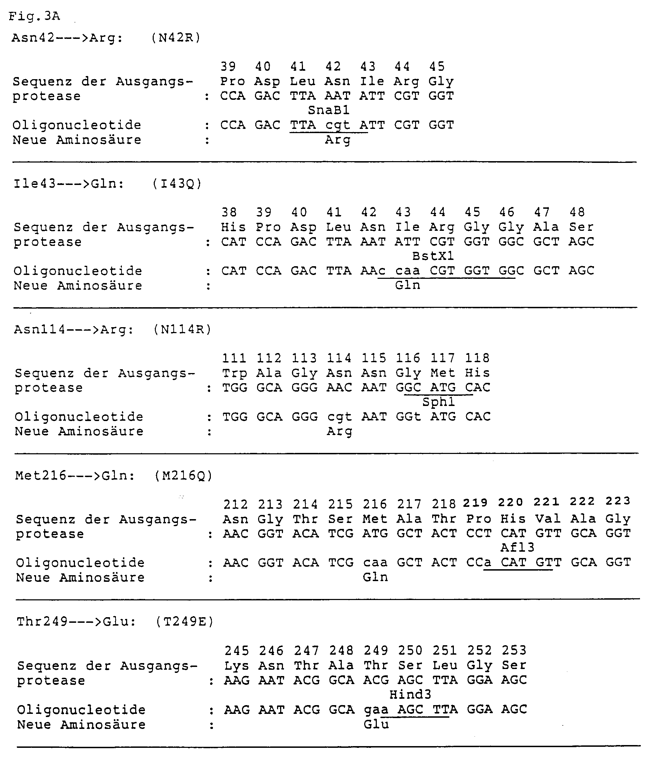

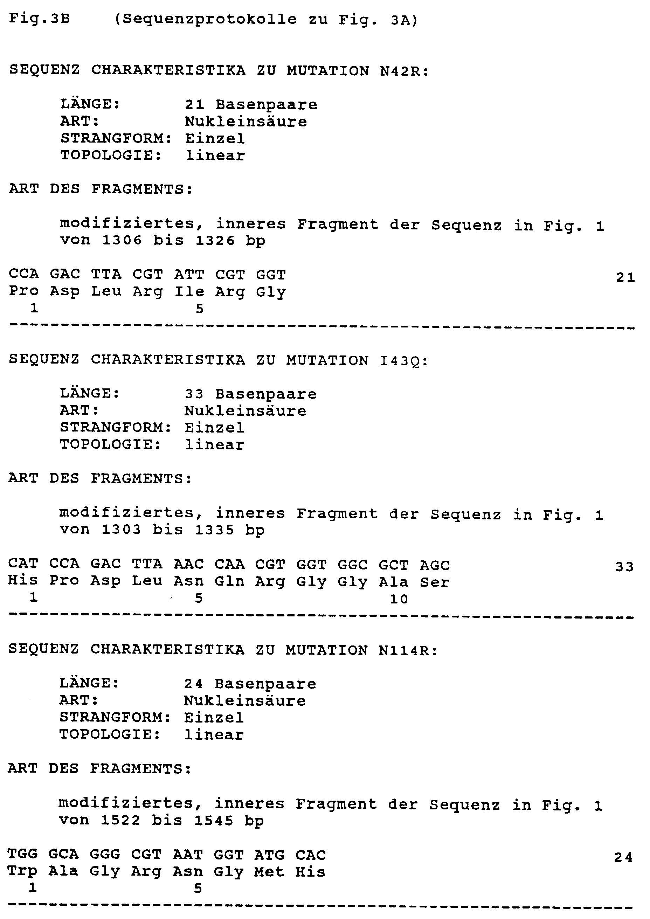

Erläuterungen zu den Figuren:
Figur 1:
   Sequenzprotokoll der DNA-Sequenz des AvaI/HindIII-Fragmentes mit dem Strukturgen der hochalkalischen Ausgangsprotease aus Bacillus alcalophilus HA1 und der Aminosäurensequenz dieser Ausgangsprotease.
Figur 2:
   Restriktionskarte des Plasmids pCLEAN4.
Figur 3A:
   DNA-Sequenzen für die zur gerichteten Mutagenese verwendeten synthetischen Oligonukleotide und Angabe eliminierter bzw. erzeugter Erkennungsstellen für einzelne Restriktionsendonukleasen; die gegenüber der ursprünglichen DNA-Sequenz der Ausgangsprotease erzeugten Nukleotidveränderungen sind durch Angabe der veränderten Nukleotide mit kleinen Buchstaben gekennzeichnet.
Figur 3B:
   Sequenzprotokolle zu den DNA-Sequenzen der Figur 3A.
Figur 4:
   Restriktionskarte des Vektors pCLMUTN1.
Figur 5:
   Restriktionskarte des Vektors pCLMUTC1.
Figur 6:
   Restriktionskarte des Vektors pUBC132
Figur 7:
   Restriktionskarte des Vektors pAL1P
Figur 8:
   Restriktionskarte der Expressionsvektoren vom Typ pAL1NC für die Expression der durch Mutation veränderten hochalkalischen Proteasen und der hochalkalischen Ausgangsprotease.
Figur 9:
   Restriktionskarte des Vektors pAL1N
Figur 10:
   Restriktionskarte des Vektors pAL1C

### Beispiel 1:

### Herstellung einer genomischen DNA-Bibliothek aus B. alcalophilus und Isolierung des Gens für die hochalkalische Ausgangsprotease

Aus dem Naturisolat Bacillus alcalophilus HA1 (hinterlegt bei der Deutschen Sammlung von Mikroorganismen unter der Nummer DSM 5466) wurde nach der Methode von Saito et al. (1963, Biochim. Biophys. Acta. 72: 619-629) chromosomale DNA isoliert und mit der Restriktionsendonuklease Sau3A partiell hydrolisiert. Die Restriktionsfragmente wurden durch Elektrophorese auf einem Agarosegel aufgetrennt und die Fragmente mit einer Größe von 3 bis 8 Kilobasen (KB) wurden isoliert.

Die isolierten und größenselektierten DNA-Fragmente aus Bacillus alcalophilus HA1 wurden mit Vektor-DNA des Plasmids pUB 110 (Gewinnung wie in Beispiel 7 beschrieben) in vitro neu kombiniert.

Hierzu wurde das Plasmid pUB110 zunächst mit der Restriktionsendonuklease BamHI restringiert und anschließend mit alkalischer Phosphatase aus Kälberdarm dephosphoryliert. Anschließend wurden 2 µg der restringierten und dephosphorylierten Vektor-DNA mit 8 µg der Bacillus alcalophilus DNA-Fragmente in einem Gesamtvolumen von 100 µl mit T4-DNA Ligase 24 h bei 16 °C inkubiert.

Mit der erhaltenen in vitro neu kombinierten DNA wurden Protoplasten des Stammes Bacillus subtilis BD224 nach der von S. Chang und N. Cohen (1979, Mol. Gen. Genet. 168: 111-115) beschriebenen Methode transformiert. Die Transformanten wurden auf Platten mit Neomycin selektiert und anschließend auf Magermilchagar überführt. Unter 13800 untersuchten Transformanten wurde eine gefunden, die durch Proteolyse des Magermilchagars einen deutlich größeren Hof bildete. Aus diesem Klon wurde die Plasmid-DNA nach Maniatis et al. isoliert. Das in diesem Plasmid enthaltene klonierte Fragment aus der B. alcalophilus-DNA hatte eine Größe von 4,1 KB und enthielt die vollständige Information für die hochalkalische Protease aus Bacillus alcalophilus HA1.

Zur Vereinfachung des weiteren Verfahrens wurde das 4,1 KB große DNA-Fragment zunächst in der Größe reduziert. Hierzu wurden die auf dem DNA-Fragment befindlichen Erkennungsstellen für Restriktionsendonukleasen durch Schneiden des Plasmids mit verschiedenen Restriktionsendonukleasen und Auftrennung der Fragmente der restringierten DNA durch Elektrophorese auf einem Agarosegel ermittelt. Es wurde ein 2,3 KB großes, durch Schneiden mit den Restriktionsendonukleasen AvaI und HindIII erhältliches DNA-Fragment ermittelt, welches die vollständige Information für die hochalkalische Protease aufwies und welches für das weitere Verfahren verwendet wurde. Hierzu wurde das obige Plasmid mit dem 4,1 KB-Fragment mit den Restriktionsendonukleasen AvaI und HindIII restringiert. Das 2,3 KB große DNA-Fragment wurde isoliert und mit dem Vektor pUB131 (Gewinnung wie in Beispiel 7 beschrieben), der zuvor ebenfalls mit AvaI und HindIII geschnitten wurde, ligiert.

Das erhaltene Plasmid, das die Bezeichnung pCLEAN4 erhielt, wurde in den Stamm B. subtilis BD224 eingebracht. Die Transformanten waren in der Lage, die hochalkalische Protease auszuscheiden, was zeigt, daß das AvaI/HindIII-Fragment das vollständige Strukturgen für die hochalkalische Protease aus B. alcalophilus HA1 enthält. Die Restriktionskarte des Plasmids pCLEAN4 ist in Fig. 2 wiedergegeben.

### Beispiel 2:

### Sequenzierung des Strukturgens für die hochalkalische Protease

Zur Herstellung von Einzelstrang-DNA des Proteasestrukturgens wurde das Plasmid pCLEAN4 mit den Restriktionsendonukleasen AvaI und HindIII geschnitten und das etwa 2,3 KB große AvaI/HindIII-DNA-Fragment (Proteasestrukturgen) in den Phagemiden pBS (+) oder pBS (-) eingebracht. Die Nukleotidsequenz des in den isolierten Einzelstrang-Phagemiden enthaltenen Proteasegens wurde nach der Dideoxy-Kettenterminator-Methode von Sanger et al. (1977, Proc. Natl. Acad. Sci. USA 74: 5463) und der Methode der basenspezifischen chemischen Spaltung des DNA-Einzelstranges nach Maxam et al. (1980, in Methods in Enzymology, Grossmann L., Moldave K., eds., Academic Press Inc., New York und London, Vol. 65, 499) bestimmt. Die ermittelte Nukleotidsequenz und die zugeordnete Aminosäurensequenz der Protease sind in Fig. 1 wiedergegeben. Der Start für die Aminosäurensequenz der reifen hochalkalischen Protease in Position 1190 der Nukleotidsequenz wurde durch Aminosäurensequenzierung des N-terminalen Endes der hochalkalischen Protease bestimmt.

### Beispiel 3:

### Herstellung mutierter DNA-Sequenzen durch gerichtete Mutagenese

Die gerichteten Mutationen wurden in DNA-Teilsequenzen des Proteasestrukturgens mit der von Kunkel, T.A. (1985, Proc. Natl. Acad. Sci. USA 82: 488-492) beschriebenen "primer extension" Technik durchgeführt. Hierzu wurden die Plasmide pCLMUTN1 (Herstellung wie in Beispiel 4 beschrieben) und pCLMUTC1 (Herstellung wie in Beispiel 5 beschrieben), die zunächst wie nachfolgend beschrieben in ihre uracylierten, einzelsträngigen Analoga umgewandelt wurden,eingesetzt. Die Ausgangsvektoren pCLMUTN1 und pCLMUTC1 enthalten nicht die gesamte DNA-Sequenz des Proteasestrukturgens aus B. alcalophilus HA1, sondern nur die N-terminale Hälfte (pCLMUTN1) oder die C-terminale Hälfte (pCLMUTC1) desselben.

Diese Vektoren sind als Abkömmlinge eines Phagemiden in gewissem Umfang zur Bildung von Einzelstrang-Vektor-DNA befähigt, die unter den hier gegebenen Bedingungen aus dem zur Replikation dienenden Wirtsorganismus ausgeschleust und isoliert werden konnte.

Jeder dieser Vektoren wurde nach Maniatis et al. (S. 250-251) mit Hilfe der CaCl₂-Methode in E. coli CJ236 als Wirtsorganismus eingebracht.

Da das Bakterium E. coli CJ236 (Uracil-N-Glycosylase-Mangelmutante) bei der Replikation von Vektoren statt Thymin das Nukleotid Uracil in die DNA-Sequenz des Vektors einbaut, wurden durch Kultivierung der vorstehenden Transformanten die Uracilhaltigen Analoga des Vektors pCLMUTN1 oder pCLMUTC1 erhalten. Diese Uracil-enthaltenden Vektoren sind von den gewöhnlichen Thymin-enthaltenden Vektoren bei in vitro-Reaktionen nicht unterscheidbar. Der Uracil-Gehalt in der Vektor-DNA stört in vitro DNA-Synthesen nicht, da Uracil weder in vitro noch in vivo mutagen ist und Uracil gleichermaßen wie Thymin codiert. Uracylierte Vektoren lassen sich vorteilhaft für die nachfolgenden in vitro Reaktionen der gerichteten Mutagenese einsetzen. Nach Beendigung der Reaktionen kann der Uracil-haltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann sowohl mit dem isolierten Enzym als auch mit einem durch Vektor-DNA transformierten E. coli-Stamm mit Uracil-N-Glycosylase-Aktivität durchgeführt werden.

Die für die gerichtete Mutagenese als Matrize benötigte, uracylierte einzelsträngige DNA der Vektoren pCLMUTN1 und pCLMUTC1 wurde hergestellt, indem mit einem der beiden Vektoren transformierte E. coli CJ236-Bakterien kultiviert wurden, die zusätzlich mit dem Helfer-Phagen M13K07 (bezogen von Bio-Rad Laboratories, Richmond, Kalifornien) infiziert wurden.

Der Helfer-Phage selbst ist kaum vermehrungsfähig und zeigt keine störende Interaktion mit der Vektor-DNA der Vektoren pCLMUTN1 oder pCLMUTC1. Seine Aufgabe besteht in der Synthese von Hüllproteinen für die gebildete uracylierte einzelsträngige Vektor-DNA. Umhüllte Einzelstrang-Vektor-DNA wird aus dem Wirtsorganismus E. coli CJ236 ausgeschleust und kann aus dem Kulturmedium isoliert werden. Durch die Mithilfe des Helfer-Phagen wird die qualitative und quantitative Ausbeute an (hier an uracylierter) Einzelstrang-Vektor-DNA wesentlich erhöht.

Die isolierten, uracylierten DNA-Einzelstrang-Vektoren pCLMUTN1 oder pCLMUTC1 wurden mit den nach Beispiel 8 hergestellten, synthetischen Oligonukleotiden hybridisiert, die eine Mutationsstelle enthielten und gleichzeitig als Primer für die nachfolgende Ergänzung zum vollständigen DNA-Doppelstrang mit Mutation dienten.

Die Synthese des zweiten DNA-Stranges wurde unter Zugabe von Nukleotiden mit T4-DNA-Polymerase und die nachfolgende Ligation des neugebildeten Stranges mit T4-DNA-Ligase durchgeführt (Kunkel et al. 1987, Methods in Enzymol. 154, 367-382). Die gebildete Doppelstrang-Vektor-DNA wurde in E. coli MC1061 transformiert und die mutierten Vektoren wurden durch Überprüfen der entsprechenden unitären Restriktionsendonukleasen-Erkennungsstellen, die mit den synthetischen Oligonukleotiden eingeführt bzw. entfernt wurden, identifiziert.

Zur Herstellung von z. B. zwei Mutationen entweder im N-terminalen oder C-terminalen Teil des Proteasestrukturgens wurde das Verfahren dieses Beispiels nach der Einführung einer ersten Mutation (Verwendung eines ersten synthetischen Oligonukleotids des Beispiels 6) in einen Teil des Proteasestrukturgens in analoger Weise unter Verwendung eines weiteren synthetischen Oligonukleotids des Beispiels 8 zur Einführung einer zweiten Mutation in diesen Teil des Proteasestrukturgens wiederholt. Man erhielt so mutierte Vektoren vom Typ pCLMUTN1 oder pCLMUTC1 mit z. B. zwei Mutationen entweder im N-terminalen oder im C-termialen Teil des Proteasestrukturgens.

### Beispiel 4:

### Konstruktion des Vektors pCLMUTN1

Das in Beispiel 1 hergestellte Plasmid pCLEAN4 wurde mit AvaI geschnitten. Die überstehenden Enden ("sticky ends") wurden unter Zugabe der benötigten Nukleotide mit Hilfe des Klenow-Fragments der E. coli DNA-Polymerase I (Maniatis et al., S. 114) zum DNA-Doppelstrang aufgefüllt. Nach anschließender Restriktion dieser DNA mit XbaI wurde das N-terminale 1618 Basenpaar (BP) umfassende Fragment des Proteasegens isoliert und in die SmaI/XbaI-Stelle von pBS kloniert. Der resultierende Vektor erhielt die Bezeichnung pCLMUTN1. Die Restriktionskarte dieses Vektors ist in Fig. 4 wiedergegeben.

### Beispiel 5:

### Konstruktion des Vektors pCLMUTC1

Das in Beispiel 1 hergestellte Plasmid pCLEAN4 wurde mit den Restriktionsendonukleasen XbaI und Asp718 geschnitten. Das 658 BP umfassende XbaI/Asp718-Doppelstrang-DNA-Fragment, welches die C-terminale Hälfte des Proteasestrukturgens umfaßt, wurde in die XbaI/Asp718-Stelle von pBS kloniert. Der resultierende Vektor erhielt die Bezeichnung pCLMUTC1. Die Restriktionskarte des Vektors ist in Fig. 5 wiedergegeben.

### Beispiel 6:

### Synthese künstlicher Oligonukleotide für die gerichtete Mutagenese

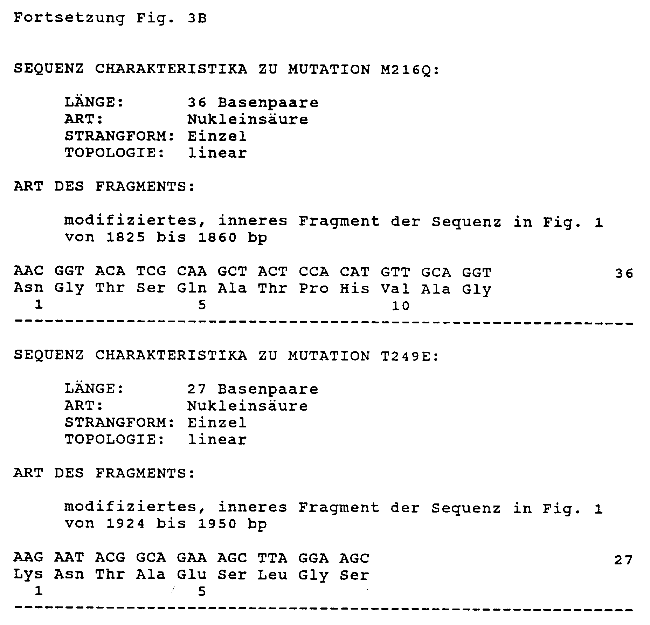

Synthetische Oligonukleotide wurden nach Beaucage S.L. und Caruthers M.H. (1981,Tetrahedron Letters 22: 1859-1862) mit β-Cyanoethyl-phosphoramidit in einem Cyclone Synthetiser (Biosearch) hergestellt. Die erhaltenen Oligonukleotide wurden gereinigt durch Elution aus Polyacrylamidgelen und anschließende Entsalzung mit Hilfe von Sephadex-G25-Säulen. Beispiele für die synthetisierten Nukleotidsequenzen und deren Eigenschaften sind in Fig. 3 wiedergegeben. Die Sequenzen der synthetischen Oligonukleotide, die im Verfahren nach Beispiel 3 zur Einführung der Mutationen in das Proteasegen dienten, waren so gewählt, daß sie die nachfolgenden Bedingungen erfüllten.
- Die DNA-Sequenz der synthetischen Oligonukleotide war zur entsprechenden Sequenz des Proteasegens noch soweit komplementär, daß ausreichende Hybridisierungsfähigkeit derselben sichergestellt war.
- Austausch eines oder mehrerer Nukleotide innerhalb des Codons, welches für die auszutauschende Aminosäure codiert, durch andere Nukleotide, so daß dieses mutierte Codon nunmehr für eine andere Aminosäure codierte (Mutation). Für die neue Aminosäure wurde dasjenige Codon eingesetzt, welches im Proteasegen für die entsprechende Aminosäure am häufigsten vorgefunden wurde.
- Austausch von weiteren Nukleotiden innerhalb anderer Codons, so daß die ursprüngliche Codierung der Aminosäure zwar erhalten blieb, aber dadurch im Proteasegen vorkommende Erkennungssequenzen für Restriktionsendonukleasen entfernt oder neue erzeugt wurden. Diese dienten im Verfahren nach Beispiel 3 zur Erleichterung des Screenings nach den Vektoren mit den mutierten DNA-Sequenzen für die neuen hochalkalischen Proteasen.

### Beispiel 7:

### Isolierung und Reinigung des Plasmids pUB110 und Konstruktion des Vektors pUB131

Aus dem Stamm Bacillus subtilis BD366 wurde nach der Methode von T.J. Gryczan et al. (1978, J. Bacterial. 134: 318-329) das Plasmid pUB110 isoliert und anschließend nach Maniatis et al. (S. 93) über Cäsiumchlorid-Dichtegradientenzentrifugation gereinigt. Der Vektor pUB110 enthält eine nur einmal vorkommende Restriktionsstelle für die Restriktionsendonukleasen BamHI und EcoRI als Marker eine DNA-Sequenz, die für Antibiotikaresistenz gegenüber Neomycin codiert, sowie für die Replikation in Bacillus-Spezies benötigte DNA-Sequenzen ("origin of replication").

Das vorstehend erhaltene Plasmid pUB110 wurde mit EcoRI und BamHI restringiert. Das kleinere Fragment (790 BP) wurde ersetzt durch einen aus 67 Basenpaaren bestehenden Polylinker, der zuvor als EcoRI/BglII-Fragment aus dem Vektor M13tg131 isoliert worden war. Der so erhaltene Vektor mit der Bezeichnung pUB131 ist somit ein Abkömmling von pUB110, bei dem das etwa 0,8 KB große EcoRI/BamHI-Fragment deletiert und dafür eine Polyklonierungsstelle eingebaut wurde.

### Beispiel 8:

### Konstruktion der Vektoren pUBC131 und pUBC132

Das Plasmid pUC18 wurde mit AatII und PvuII geschnitten. Das 1990 Basenpaar große Fragment mit dem β-Lactamase-Gen und dem E. coli "origin of replication" wurde isoliert. Die überstehenden Enden ("sticky ends") wurden unter Zugabe der benötigten Nukleotide mit Hilfe des Klenow-Fragmentes der E. coli DNA-Polymerase I (Maniatis et al., S. 114) zum DNA-Doppelstrang aufgefüllt. Das Fragment wurde anschließend in die SnaBI-Stelle des nach Beispiel 7 erhaltenen Vektors pUB131 eingebaut, wodurch der Vektor mit der Bezeichnung pUBC131 erhalten wurde.

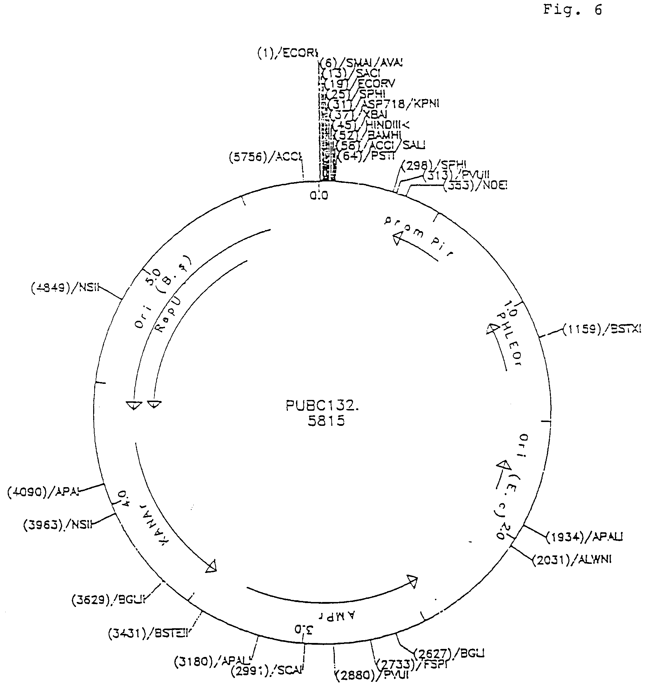

Das 2187 BP EcoRI/BglII-Fragment des vorstehend erhaltenen Vektors pUBC131 wurde in die EcoRI/BamHI-Stelle von pBS (+) subkloniert, wobei man den Vektor mit der Bezeichnung pBSREPU erhielt. Anschließend wurde die NcoI- bzw. StyI-Erkennungsstelle, die in der DNA-Sequenz für das repU-Polypeptid im Vektor pBSREPU vorhanden ist (I. Maciag et al. 1988, Mol. Gen. Genet. 212: 232-240), durch gerichtete Mutagenese eliminiert, indem die Nukleotidsequenz CCA TGG durch die Nukleotidsequenz CCG TGG (beide Nukleotidsequenzen codieren für die Aminosäurenfolge Tryptophan-Prolin) ersetzt wurde. Die Durchführung war analog der Verfahrensweise des Beispiels 3. Hierzu wurde uracylierte einzelsträngige DNA des Vektors pBSREPU als Matrize für die gerichtete Mutation zur Eliminierung der NcoI- bzw. StyI-Erkennungsstelle hergestellt. Anschließend wurde diese Matrize analog zur im Beispiel 3 beschriebenen "primer extension"-Technik unter Verwendung des folgenden synthetischen Oligonukleotids (hergestellt und gereinigt analog zum Verfahren zur Herstellung der synthetischen Oligonukleotide des Beispiels 6) 
NcoI/StyI
Pro Trp
Originäre repU-Sequenz   : AAA GTG AGA CCA TGG AGA GAA AA
Synthetische repU-Sequenz: AAA GTG AGA CCg TGG AGA GAA AA
zum DNA-Doppelstrang-Vektor ergänzt und durch Transformation und Kultivierung von E. coli MC1061 die nunmehr NcoI- bzw. StyI-Erkennungsstellen-freien Vektoren isoliert. Das 1726 BP EcoRI/ApaI-Fragment des isolierten Vektors wurde in die EcoRI/ApaI-Stelle von pUBC131 eingeführt. Der neue Vektor, dessen Restriktionskarte in Fig. 6 wiedergegeben ist, erhielt die Bezeichnung pUBC132.

### Beispiel 9:

### Konstruktion des Plasmids pAL1P

Das Plasmid pAL1P wurde hergestellt durch Ligation der folgenden drei Elemente:
- das 1201 Basenpaar große AvaI/NcoI-Fragment von pCLEAN4; das Fragment enthält den Promotor und die Prepro-Region der hochalkalischen Ausgangsprotease.
- der folgende synthetische Linker mit einzelnen Erkennungsstellen für die Restriktionsendonukleasen NcoI, XbaI und Asp718, die die Einführung der mutierten N-terminalen bzw. C-terminalen Hälften des Proteasegens aus den mutierten Vektoren pCLMUT1 bzw. pCLMUTC1 oder die Einführung des gesamten Gens der Ausgangsprotease aus dem Plasmid pCLEAN4 ermöglichen; 
5' - CCATGGTCTAGAGGTACCA - 3'
3' - CAGATCTCCATGGTTCGAA - 5'
^ ^ ^ ^
NcoI XbaI Asp718
HindII
 Der vorstehende doppelsträngige synthetische Linker mit überstehenden 5'-Enden wurde hergestellt, indem man zunächst die beiden Einzelstrang-DNA-Sequenzen analog zur Synthese der synthetischen Oligonukleotide in Beispiel 6 jeweils für sich herstellte und reinigte. Die so erhaltenen Einzelstränge wurden nachfolgend miteinander zum Doppelstrang hybridisiert.
- Das 5776 Basenpaar große AvaI/HindIII-Fragment aus dem in Beispiel 8 hergestellten Vektor pUBC132; dieses Fragment enthält DNA-Sequenzen zur Replikation und selektierbare Marker in E. coli, sowie DNA-Sequenzen zur Replikation und selektierbare Marker in B. subtilis, B. licheniformis und B. alcalophilus.

Die Konstruktion des Vektors pAL1P wurde in E. coli MC1061 durchgeführt und der Vektor aus Ampicillin-resistenten E. coli-Transformanten isoliert. Die Restriktionskarte des erhaltenen Vektors ist in Fig. 7 wiedergegeben.

### Beispiel 10:

### Konstruktion der Plasmide pAL1N und pAL1C

Die Konstruktion der Vektoren pAL1N und pAL1C wurde in E. coli MC1061 durchgeführt, wobei die Vektoren aus Ampicillin-resistenten E. coli-Transformanten isoliert wurden.

Das Plasmid pAL1N wurde konstruiert, indem zunächst der in Beispiel 1 erhaltene Vektor pCLEAN4 mit den Restriktionsendonukleasen NcoI und XbaI geschnitten und das erhaltene NcoI/XbaI-Fragment anschließend in die NcoI/XbaI-Stelle des Vektors pAL1P (hergestellt nach Beispiel 9) kloniert wurde. Der hergestellte Vektor enthielt den N-terminalen Teil der DNA-Sequenz, die für das reife Enzym codiert, und die regulatorischen Elemente für die Transkription und Translation der hochalkalischen Protease, sowie die Signal-Sequenz und die Processing-Sequenz.

Das Plasmid pAL1C wurde konstruiert, indem zunächst der in Beispiel 1 erhaltene Vektor pCLEAN4 mit den Restriktionsendonukleasen XbaI und Asp718 geschnitten und das erhaltene XbaI/Asp718-Fragment in die XbaI/Asp718-Stelle des Vektors pAL1P (hergestellt nach Beispiel 9) kloniert wurde. Der hergestellte Vektor enthielt den C-terminalen Teil der DNA-Sequenz, die für die reife Protease codiert, und die regulatorischen Elemente für die Transkription und Translation der hochalkalischen Protease, sowie die Signal-Sequenz und die Processing-Sequenz.

### Beispiel 11:

### Konstruktion der Expressionsvektoren pAL1NC

Es wurden Expressionsvektoren mit Mutationen im C-terminalen Teil der Protease-DNA-Sequenz, Expressionsvektoren mit Mutationen im N-terminalen Teil der Protease-DNA-Sequenz, Expressionsvektoren mit Mutationen im N- und C-terminalen Teil der DNA-Sequenz und zu Vergleichszwecken auch Expressionsvektoren ohne Mutationen in der Protease-DNA-Sequenz hergestellt.

### A. Expressionsvektor mit Mutationen im N-terminalen Teil der DNA-Sequenz der Protease

Der nach Beispiel 3 durch gerichtete Mutagenese erhaltene mutierte Vektor pCLMUTN1 wurde mit den Restriktionsendonukleasen NcoI und XbaI geschnitten. Das isolierte 411 Basenpaar große NcoI/XbaI-Fragment (mutierter N-terminaler Teil des Proteasestrukturgens mit jeweils den Mutationen N42R, N114R, I43Q, N42R/N114R und I43Q/N114R, N42R/I43Q, N42R/I43Q/N114R) wurde in die NcoI/XbaI-Stelle des nach Beispiel 15 erhaltenen Plasmides pAL1C kloniert. Der jeweils erhaltene Vektor stellt einen vollständigen Expressionsvektor mit geeignetem Leseraster zur Expression einer mutierten Protease dar. Die Vektoren wurden folgendermaßen bezeichnet:
- pALN42R: = Expressionsvektor für die Protease mit der Mutation N42R;
- pALI43Q: = Expressionsvektor für die Protease mit der Mutation I43Q;
- pALN114R: = Expressionsvektor für die Protease mit der Mutation N114R;
- pALN42R/N114R: = Expressionsvektor für die Protease mit den Mutationen N42R/N114R;
- pALI43Q/N114R: = Expressionsvektor für die Protease mit den Mutationen I43Q/N114R.
- pALN42R/I43Q: = Expressionsvektor für die Protease mit den Mutationen N42R/I43Q.
- pALN42R/I43Q/N114R: = Expressionsvektor für die Protease mit den Mutationen N42R/I43Q/N114R.

### B. Expressionsvektor mit Mutationen im C-terminalen Teil der DNA-Sequenz der Protease

Der nach Beispiel 3 durch gerichtete Mutagenese erhaltene mutierte Vektor pCLMUTC1 wurde mit den Restriktionsendonukleasen XbaI und Asp718 geschnitten. Das isolierte 609 Basenpaar große XbaI/Asp718-Fragment (mutierter C-terminaler Teil des Proteasestrukturgens mit jeweils den Mutationen M216Q, T249E und M216Q/T249E) wurde in die XbaI/Asp718-Stelle des nach Beispiel 10 erhaltenen Plasmides pAL1N kloniert. Der jeweils erhaltene Vektor stellt einen vollständigen Expressionsvektor mit geeignetem Leseraster zur Expression einer mutierten Protease dar. Die Vektoren wurden folgendermaßen bezeichnet:
- paLM216Q: = Expressionsvektor für die Protease mit der Mutation M216Q;
- pALT249E: = Expressionsvektor für die Protease mit den Mutationen T249E;
- pALM216Q/T249E: = Expressionsvektor für die Protease mit den Mutationen M216Q/T249E.

### C. Expressionsvektor mit Mutationen im C-terminalen und N-terminalen Teil der Protease-DNA-Sequenz

Der in diesem Beispiel unter A. hergestellte Expressionsvektor pALN114R und der unter B. hergestellte Expressionsvektor pALM216Q wurden jeweils mit den Restriktionsendonukleasen XbaI und AvaI geschnitten. Das 1612 Basenpaar große Fragment des Plasmids pALN114R wurde mit dem 6388 Basenpaar großen Fragment des Plasmids pALM216Q ligiert. Das resultierende Plasmid erhielt die Bezeichnung pALN114R/M216Q.

Analog wurden die folgenden Expressionsvektoren hergestellt:
pALN114R/M216Q
pALI43Q/M216Q
pALN42R/M216Q
pALI43Q/M216Q/T249E
pALI43Q/N114R/T249E
pALI43Q/N114R/M216Q/T249E
pALN42R/I43Q/N114R/M216Q/T249E
pALN114R/T249E
pALI43Q/N114R/M216Q
pALN114R/M216S
pALI43Q/M216S/T249E
pALN42R/M216A
pALN42R/M216A/T249E.

### D. Expressionsvektor mit der nicht mutierten DNA-Sequenz der Ausgangsprotease

Der Expressionsvektor mit dem nicht mutierten Ausgangsstrukturgen der Protease wurde erhalten, indem entweder das 411 Basenpaar große, nicht-mutierte NcoI/XbaI-Fragment aus dem nach Beispiel 1 erhaltenen Plasmid pCLEAN4 in die NcoI/XbaI-Stelle des nach Beispiel 10 erhaltenen Plasmides pAL1C kloniert wurde; oder indem das 609 Basenpaar große XbaI/Asp718-Fragment aus dem nach Beispiel 1 erhaltenen Plasmid pCLEAN4 in die XbaI/Asp718-Stelle des nach Beispiel 10 erhaltenen Plasmides pAL1N kloniert wurde. Die so erhaltenen Vektoren sind vollständige Expressionsvektoren mit geeignetem Leseraster zur Expression der nicht mutierten hochalkalischen Ausgangsprotease.

Die Restriktionskarte dieser Vektoren des Typs pAL1NC ist in Fig. 8 wiedergegeben.

### Beispiel 12:

### Herstellung der durch Mutation veränderten hochalkalischen Proteasen und zu Vergleichszwecken auch der Ausgangsprotease

50 ml Vorkulturmedium (20 g Tryptone, 10 g Hefe-Extrakt,
5 g NaCl, 75 g lösliche Stärke, 10 ml Maisquellwasser pro Liter) wurden mit einer Kolonie der zu testenden Stämme (jeweils mit einem der nach Beispiel 11 hergestellten Vektoren pAL1NC transformierte B. subtilis BD224) beimpft. Die Kultur wurde für 16 h bei 37 °C und 250 Upm inkubiert. Mit 2,5 ml dieser Kultur wurden 50 ml Hauptkulturmedium (30 g Sojamehl, 90 g Kartoffelstärke, 10 g Na-Caseinat und 10 ml Maisquellwasser pro Liter) beimpft. Die Hauptkultur wurde unter den gleichen Bedingungen wie die Vorkultur inkubiert. Nach 72 h wurden die Kulturen zentrifugiert. Die gebildeten Proteasen wurden aus den Kulturbeständen mit Aceton gefällt und anschließend wie folgt gereinigt: Kationenaustauscher Mono S, FPLC; Elution mit ansteigendem Gradient 20 mMol bis 200 mMol Ammoniumacetat, pH = 6.

## Patentansprüche

1. Hochalkalische Protease, die eine Aminosäuresequenz aufweist, welche wenigstens 80 % Homologie zu der in Fig. 1 angegebenen Aminosäuresequenz besitzt und sich von dieser in zwei, drei vier oder fünf der Positionen 42, 43, 114, 216, 249 oder den dazu homologen Positionen unterscheidet, wobei bei einem Austauch an den betreffenden Positionen
die sich in Position 42 befindliche Aminosäure gegen Arginin,
die sich in Position 43 befindliche Aminosäure gegen Glutamin,
die sich in Position 114 befindliche Aminosäure gegen Arginin,
die sich in Position 216 befindliche Aminosäure gegen Serin, Glutamin oder Alanin,
die sich in Position 249 befindliche Aminosäure gegen Glutaminsäure ausgetauscht ist.

2. Hochalkalische Protease nach Anspruch 1, dadurch gekennzeichnet, daß sie eine Aminosäurensequenz aufweist, welche mindestens 90 %, vorzugsweise mindestens 95 %, Homologie zu der in Fig. 1 angegebenen Aminosäurensequenz besitzt.

3. Hochalkalische Protease nach Anspruch 1 oder 2, dadurch gekennzeichnet, daß sie eine Aminosäurensequenz aufweist, in welcher die sich in Position 216 befindliche Aminosäure gegen Serin, Glutamin oder Alanin und entweder die sich in Position 43 befindliche Aminosäure gegen Glutamin oder die sich in Position 114 befindliche Aminosäure gegen Arginin oder die sich in Position 249 befindliche Aminosäure gegen Glutaminsäure ausgetauscht ist.

4. Hochalkalische Protease nach Anspruch 1 oder 2, dadurch gekennzeichnet, daß sie eine Aminosäurensequenz aufweist, in welcher die sich in Position 216 befindliche Aminosäure gegen Serin, Glutamin oder Alanin und die sich in Position 114 befindliche Aminosäure gegen Arginin und entweder die sich in Position 42 befindliche Aminosäure gegen Arginin oder die sich in Position 43 befindliche Aminosäure gegen Glutamin ausgetauscht ist.

5. Hochalkalische Protease nach Anspruch 1 oder 2, dadurch gekennzeichnet, daß sie eine Aminosäurensequenz aufweist, in welcher in die sich in Position 43 befindliche Aminosäure gegen Glutamin und die sich in Position 249 befindliche Aminosäure gegen Glutaminsäure und entweder die sich in Position 114 befindliche Aminosäure gegen Arginin oder die sich in Position 216 befindliche Aminosäure gegen Serin, Glutamin oder Alanin ausgetauscht ist.

6. Hochalkalische Protease nach Anspruch 1 oder 2, dadurch gekennzeichnet, daß sie eine Aminosäurensequenz aufweist, in welcher die sich in Position 43 befindliche Aminosäure gegen Glutamin und die sich in Position 114 befindliche Aminosäure gegen Arginin und die sich in Position 249 befindliche Aminosäure gegen Glutaminsäure und die sich in Position 216 befindliche Aminosäure gegen Serin, Glutamin oder Alanin ausgetauscht ist.

7. Hochalkalische Protease nach einem der Ansprüche 3 bis 6, dadurch gekennzeichnet, daß die sich in Position 216 befindliche Aminosäure gegen Glutamin ausgetauscht ist.

8. Hochalkalische Protease nach Anspruch 1 oder 2, dadurch gekennzeichnet, daß sie eine Aminosäurensequenz aufweist, in welcher die sich in Position 114 befindliche Aminosäure gegen Arginin und entweder die sich in Position 43 befindliche Aminosäure gegen Glutamin oder die sich in Position 249 befindliche Aminosäure gegen Glutaminsäure ausgetauscht ist.

9. DNA-Sequenz, codierend für eine hochalkalische Protease mit einer Aminosäurensequenz, welche wenigstens 80 % Homologie zu der in Fig. 1 angegebenen Aminosäurensequenz besitzt und sich von dieser in zwei, drei, vier oder fünf der Positionen 42, 43, 114, 216, 249 oder den dazu homologen Positionen unterscheidet, wobei bei einem Austauch an den betreffenden Positionen
die sich in Position 42 befindliche Aminosäure gegen Arginin,
die sich in Position 43 befindliche Aminosäure gegen Glutamin,
die sich in Position 114 befindliche Aminosäure gegen Arginin,
die sich in Position 216 befindliche Aminosäure gegen Serin, Glutamin oder Alanin,
die sich in Position 249 befindliche Aminosäure gegen Glutaminsäure ausgetauscht ist.

10. DNA-Sequenz nach Anspruch 9, dadurch gekennzeichnet, daß sie für eine Aminosäurensequenz codiert, in welcher die Aminosäuren gemäß einem der Ansprüche 3 bis 8 ausgetauscht sind.

11. Zusammensetzungen zum Waschen, Reinigen oder Geschirrspülen, enthaltend eine hochalkalische Protease gemäß einem der Ansprüche 1 bis 8.

12. Zusammensetzungen nach Anspruch 11, dadurch gekennzeichnet, daß sie die hochalkalische Protease in Gegenwart eines oder mehrerer von in Wasch- und Reinigungsmitteln üblichen Enzymen aus der Gruppe der Proteasen, Lipasen, Pektinasen, Amylasen, Nukleasen, Oxidoreduktasen und Cellulasen enthalten.

13. Zusammensetzungen nach Anspruch 12, enthaltend Buildersubstanzen, Bleichmittel sowie gegebenenfalls weitere als Waschmittelbestandteile übliche Zusatz- und Hilfsstoffe.

14. Verfahren zur Herstellung einer hochalkalischen Protease mit der in Anspruch 1 angegebenen Aminosäurensequenz, dadurch gekennzeichnet, daß man einen transformierten Mikroorganismus kultiviert, der eine DNA-Sequenz enthält, die für eine Aminosäurensequenz codiert, welche wenigstens 80 % Homologie zu der in Fig. 1 angegebenen Aminosäurensequenz besitzt und sich von dieser in zwei, drei, vier oder fünf der Positionen 42, 43, 114, 216, 249 oder den dazu homologen Positionen unterscheidet, wobei bei einem Austausch an den betreffenden Positionen
die sich in Position 42 befindliche Aminosäure gegen Arginin,
die sich in Position 43 befindliche Aminosäure gegen Glutamin,
die sich in Position 114 befindliche Aminosäure gegen Arginin,
die sich in Position 216 befindliche Aminosäure gegen Serin, Glutamin oder Alanin,
die sich in Position 249 befindliche Aminosäure gegen Glutaminsäure ausgetauscht ist,
und aus dem Kulturmedium die gebildete hochalkalische Protease isoliert.
